# EUROPEAN PATENT APPLICATION

(11) **EP 4 331 671 A1**
(43) Date of publication of application: **06.03.2024**
(21) Application number: 22795407.0
(22) Date of filing: 25.03.2022
(51) Int. Cl.: A61P 25/22, A61P 43/00, A61K 36/07, A61K 31/4172, A23L 33/175

(54) **ANTI-ANXIETY COMPOSITION**

(30) Priority: 26.04.2021 JP 2021074317
(71) Applicant: Suntory Holdings Limited, Osaka 530-8203 (JP)
(72) Inventor: KATSUBE, Makoto, Soraku-gun, Kyoto 619-0284 (JP); WATANABE, Hiroshi, Soraku-gun, Kyoto 619-0284 (JP); MURAYAMA, Norihito, Soraku-gun, Kyoto 619-0284 (JP)
(74) Representative: Vossius & Partner Patentanwälte Rechtsanwälte mbB
(86) International application number: PCT/JP2022/014237
(87) International publication number: WO 2022/230489

(57) **Abstract**

The present invention aims to provide an anxiolytic composition for humans and a method of preventing or reducing an anxiety symptom. The present invention relates to an anxiolytic composition for humans, containing L-ergothioneine or a salt thereof as an active ingredient.

## Description

### TECHNICAL FIELD

The present invention relates to an anxiolytic composition. The present invention also relates to, for example, a method of preventing or reducing an anxiety symptom.

### BACKGROUND ART

Anxiety is an unpleasant feeling such as vague fear without an identifiable object. When anxiety continues for some reason, such anxiety may interfere with daily life and social life. Anxiety symptoms are common in those with mental disorders such as anxiety disorders, but ones not diagnosed with mental illness may sometimes feel anxiety due to stress or the like in daily life.

Ergothioneine is an amino acid found in mushrooms and the like. L-ergothioneine is present in nature. According to Patent Literature 1, mice fed with golden/yellow oyster mushroom powder containing L-ergothioneine significantly shortened motionless time compared to mice fed with regular diet in the forced swim test and tail suspension test.

### CITATION LIST

### - Patent Literature

Patent Literature 1: JP 2014-193844 A

### SUMMARY OF INVENTION

### - Technical Problem

Anxiolytics may be used to treat prolonged anxiety that is interfering with daily life, for example. However, anxiolytics have risk of side effects, so that a safer substance having an anxiolytic action is desired. According to Patent Literature 1, depressive symptoms were evaluated using as an index the motionless time of mice in the forced swim test and tail suspension test, and L-ergothioneine was confirmed to have an antidepressant effect. However, Patent Literature 1 describes the antidepressant effect on animal models of depression and nowhere studies the anxiolytic effect of L-ergothioneine on humans. Whether ingestion of L-ergothioneine by a human prevents or reduces anxiety symptoms is difficult to predict from the shortened motionless time of mice in the forced swim test and tail suspension test.

The present invention aims to provide an anxiolytic composition for humans. The present invention also aims to provide a method of preventing or reducing an anxiety symptom.

### - Solution to Problem

As a result of extensive studies on the above issue, the present inventors found that the anxiolytic effect can be obtained by ingestion of L-ergothioneine.

Specifically, the present invention relates to, but is not limited to, an anxiolytic composition and the like described below.
(1) An anxiolytic composition for humans, containing L-ergothioneine or a salt thereof as an active ingredient.
(2) The composition according to (1) above, wherein the composition reduces glutamate receptor activation.
(3) The composition according to (1) or (2) above, wherein the composition is an oral composition.
(4) The composition according to any one of (1) to (3) above, wherein the composition is a food or beverage.
(5) The composition according to any one of (1) to (4) above, wherein an amount of L-ergothioneine or a salt thereof in the composition per adult daily intake is 2 to 50 mg in terms of L-ergothioneine.
(6) A method of preventing or reducing an anxiety symptom, including administering L-ergothioneine or a salt thereof to a human.
(7) Use of L-ergothioneine or a salt thereof for preventing or reducing a human anxiety symptom.

### - Advantageous Effects of Invention

The present invention can provide an anxiolytic composition for humans. The present invention can provide a method of preventing or reducing an anxiety symptom.

### BRIEF DESCRIPTION OF DRAWINGS

FIG. 1 is a graph showing changes in state anxiety score (Δ in state anxiety (points)) in a test food group and a control food group.
FIG. 2 is a graph showing glutamate measurements identified by measuring the blood metabolome at the time of pre-testing and at the time of testing on week 4.
FIG. 3 shows results of evaluation of activity of L-ergothioneine to inhibit binding of substrates to nicotinic acetylcholine receptors (nAChR).
FIG. 4 shows results of evaluation of activity of L-ergothioneine to inhibit binding of substrates to α3β4 nicotinic acetylcholine receptors based on five concentrations.

### DESCRIPTION OF EMBODIMENTS

The anxiolytic composition of the present invention is an anxiolytic composition for humans, containing L-ergothioneine or a salt thereof as an active ingredient. Hereinafter, the anxiolytic composition of the present invention is sometimes simply referred to as "the composition of the present invention".

L-ergothioneine is one of the amino acids. The salt of L-ergothioneine is not limited as long as it is a pharmacologically acceptable salt or a dietary acceptable salt, and it may be either an acid salt or a basic salt. Examples of the acid salt include inorganic acid salts such as hydrochloride, sulfate, nitrate, and phosphate, and organic acid salts such as acetate, citrate, maleate, malate, oxalate, lactate, succinate, fumarate, and propionate. Examples of the basic salt include alkali metal salts such as sodium salt and potassium salt, and alkaline earth metal salts such as calcium salt and magnesium salt.

L-ergothioneine or a salt thereof that can be used may be a chemically synthesized product or a purified extract of a natural product. L-ergothioneine is abundantly present in golden/yellow oyster mushrooms (binomial name: *Pleurotus cornucopiae* var. *citrinopileatus*) belonging to the genus Pleurotus of the family Pleurotaceae. L-ergothioneine is also present in mushrooms such as common mushrooms (binomial name: *Agaricus bisporus*) including white mushroom, cremini mushroom, and portabella mushroom; grey oyster mushroom (binomial name: *Pleurotus ostreatus*), shiitake (binomial name: *Lentinula edodes*)*,* hen-of-the-wood (binomial name: *Grifola Frondosa*)*,* reishi mushuroom (binomial name: *Ganoderma lucidum*)*,* lion's mane mushroom (binomial name: *Hericium erinaceus*)*,* Yanagi-matsutake (binomial name: *Agrocybe aegerita*)*,* girolle (binomial name: *Cantharellus cibarius*), porcini (binomial name: *Boletus edulis*)*,* and common morel (binomial name: *Morchella esculenta*)*.* When L-ergothioneine is obtained from a natural product, preferably, it is extracted from a golden/yellow oyster mushroom, for example. L-ergothioneine or a salt thereof can also be produced by microbial fermentation. An extract containing L-ergothioneine or a salt thereof produced by microbial fermentation or a purified product thereof may be used. L-ergothioneine can be extracted and purified from natural products by known methods. L-ergothioneine or a salt thereof may be in an isolated form.

L-ergothioneine or a salt thereof is present in natural products and food and beverages, and it is a compound that has been consumed. Thus, continuous ingestion of L-ergothioneine or a salt thereof, for example, is less likely to cause problems in terms of safety.

The term "anxiolytic" includes at least one of prevention of anxiety symptoms and reduction in anxiety symptoms. The anxiolytic composition of the present invention can be used to prevent or reduce human anxiety symptoms and can be preferably used to reduce the human anxiety symptoms. Examples of anxiety symptoms include anxiety symptoms caused by stress, such as temporary (short term) anxiety symptoms caused by stress. In one embodiment, the composition of the present invention can be preferably used, for example, to prevent or reduce anxiety symptoms of a human feeling stressed and can be more preferably used to reduce the symptoms. Stress may be mental (psychological) stress.

Anxiety is classified into two types: state anxiety and trait anxiety. The term "state anxiety" refers to temporary anxiety that arises in a particular situation. Generally, a higher stress level or a longer stress exposure time results in a higher state anxiety. Temporary anxiety symptoms caused by stress include anxiety symptoms of state anxiety. The term "trait anxiety" does not refer to temporary anxiety that arises in a particular situation as in state anxiety but refers to a tendency to anxiety, which is derived from characteristics of a subject, for example. The composition of the present invention is preferably used to prevent or reduce anxiety symptoms of human state anxiety.

The term "anxiety symptoms" refers to emotional symptoms of anxiety or mental (cognitive) symptoms of anxiety.

Examples of emotional symptoms of anxiety include feelings of anxiety, fear, tension, worries, heartache, and loneliness. Emotional symptoms of anxiety may include one or more of these symptoms. Examples of mental (cognitive) symptoms of anxiety include poor memory, lethargy, nervousness, distress, mental agitation, and decreased positive thinking. Mental symptoms of anxiety may include one or more of these symptoms.

In one embodiment, the composition of the present invention can be used to prevent or reduce one or more anxiety symptoms, such as feelings of anxiety, fear, tension, worries, heartache, loneliness, poor memory, lethargy, nervousness, distress, mental agitation, and decreased positive thinking. In one embodiment, the composition of the present invention is preferably used to prevent or reduce (preferably reduce) temporary anxiety symptoms caused by stress or of humans feeling stressed, such as tension, fear, heartache, loneliness, decreased positive thinking, nervousness, distress, and mental agitation.

Human anxiety symptoms can be evaluated using State Trait Anxiety Inventory (STAI), for example. In Examples (described later), anxiety symptoms were evaluated using the State Trait Anxiety Inventory (STAI). The State Trait Anxiety Inventory (STAI Y-1) has 20 items including, "I feel calm", "I feel secure", "I am tense", "I am strained", "I feel at ease", "I feel upset", "I am presently worrying over possible misfortunes", "I feel satisfied", "I feel frightened", "I feel comfortable", "I feel self-confident", "I feel nervous", "I am jittery", "I feel indecisive", "I am relaxed", "I feel content", "I am worried", "I feel confused", "I feel steady", and "I feel pleasant", and each item is evaluated on a four-point scale from "Almost never" to "Almost always". In one embodiment, the anxiolytic composition of the present invention can be used to prevent or reduce anxiety symptoms of state anxiety evaluated by the State Trait Anxiety Inventory (STAI).

As shown in Examples (described later), ingestion of L-ergothioneine reduced scores of state anxiety evaluated by the State Trait Anxiety Inventory (STAI). This indicates that ingestion of L-ergothioneine reduced anxiety symptoms. L-ergothioneine or a salt thereof has an anxiolytic action and can be used as an active ingredient for preventing or reducing anxiety symptoms. Ingestion of L-ergothioneine also reduced the blood glutamate concentration when compared to no ingestion. Glutamate is an excitatory neurotransmitter in the central nervous system. It has been reported that psychological stress increases glutamate signaling in the brain. A reduction in glutamate receptor activation is expected to result in an anti-mental stress effect. A reduction in blood glutamate concentration enables a reduction in glutamate receptor activation.

L-ergothioneine has an action to inhibit binding of substrates to nicotinic acetylcholine receptors such as α3β4 nicotinic acetylcholine receptors and α2α4 nicotinic acetylcholine receptors. For example, α3β4 nicotinic acetylcholine receptors are expressed in the brain, and inhibition of binding of substrates to the receptors reduces glutamate signaling in the brain. Attenuation of glutamate signaling in the brain by a reduction in glutamate receptor activation or the like results in an anxiolytic effect.

The mechanism of how L-ergothioneine demonstrated the anxiolytic action is uncertain, but presumably, the effect was demonstrated by reducing glutamate receptor activation through the action to reduce the blood glutamate concentration and/or the action to inhibit nicotinic acetylcholine receptors (e.g., α3β4 nicotinic acetylcholine receptors). The composition of the present invention can reduce glutamate receptor activation and can be used for such a purpose.

In one embodiment, the anxiolytic composition of the present invention can be used to prevent or ameliorate a condition or disease which can be effectively prevented or ameliorated by preventing or reducing anxiety symptoms. Examples of such a condition or disease include anxiety disorder and adjustment disorder.

Prevention encompasses preventing the onset, delaying the onset, reducing the incidence, reducing the onset risk, and the like. Amelioration encompasses helping a subject recover, alleviating symptoms, reducing the frequency of symptoms, favorably changing symptoms, delaying or preventing the progress, and the like.

The composition of the present invention is applicable for therapeutic use (medical use) and non-therapeutic use (non-medical use). The "non-therapeutic" is a concept that does not include medical activities, i.e., a concept that does not include methods of surgery, therapy, or diagnosis of humans.

The anxiolytic composition of the present invention can be provided as an agent or the like, for example, but it is not limited thereto. The agent can be directly provided as a composition or can be provided as a composition containing the agent. In one embodiment, the anxiolytic composition of the present invention for humans can also be referred to as an anxiolytic for humans.

In order to sufficiently obtain the effect of the present invention, preferably, the composition of the present invention is an oral composition. The oral composition may be a food or beverage, an oral pharmaceutical product, or an oral quasi-pharmaceutical product, preferably a food or beverage or an oral pharmaceutical product, still more preferably a food or beverage.

The composition of the present invention may contain optional additives and optional components in addition to L-ergothioneine or a salt thereof, as long as the effect of the present invention is not impaired. Such additives and components can be selected according to the composition form or the like. Those that can be generally used in foods, beverages, pharmaceutical products, quasi-pharmaceutical products, and the like can be used. When the composition of the present invention is provided as a food or beverage, a pharmaceutical product, a quasi-pharmaceutical product, or the like, the production method is not limited, and any common method can be used for production.

For example, when the composition of the present invention is provided as a food or beverage, various types of food or beverages can be provided by adding a component usable in food or beverages (e.g., a food material or an optional food additive) to L-ergothioneine or a salt thereof. Non-limiting examples of the food or beverages include general foods and beverages, health foods, health beverages, foods with function claims, foods for specified health uses, dietary supplements, and foods and beverages for the sick. The health foods, foods with function claims, foods for specified health uses, dietary supplements, and the like can be used, for example, in various forms of preparations such as fine granules, tablets, granules, powders, capsules, chewable tablets, syrups, liquids, and liquid foods.

When the composition of the present invention is provided as a pharmaceutical product or a quasi-pharmaceutical product, for example, a pharmacologically acceptable carrier, an optional additive, or the like can be added to L-ergothioneine or a salt thereof to provide pharmaceutical products or quasi-pharmaceutical products in various dosage forms. Such a carrier, an additive, or the like may be any pharmacologically acceptable one that can be used in pharmaceutical products or quasi-pharmaceutical products. Examples thereof include excipients, binders, disintegrants, lubricants, antioxidants, and colorants. One or more of these can be used. The administration form of pharmaceutical products or quasi-pharmaceutical products may be oral or parenteral. Oral administration is preferred in order to obtain the effect of the present invention more sufficiently. When the composition of the present invention is provided as a pharmaceutical product or a quasi-pharmaceutical product, preferably, it is an oral pharmaceutical product or an oral quasi-pharmaceutical product. Examples of dosage forms for oral administration include liquids, tablets, powders, fine granules, granules, sugar-coated tablets, capsules, suspensions, emulsions, and chewable tablets. Examples of dosage forms for parenteral administration include injection and infusion.

The amount of L-ergothioneine or a salt thereof in the composition of the present invention is not limited and can be set according to the composition form and the like.

The amount of L-ergothioneine or a salt thereof, for example, in terms of L-ergothioneine, in the composition of the present invention is preferably 0.0001 wt% or more, more preferably 0.001 wt% or more and is preferably 90 wt% or less, more preferably 50 wt% or less. In one embodiment, the amount of L-ergothioneine or a salt thereof in terms of L-ergothioneine in the composition is preferably 0.0001 to 90 wt%, more preferably 0.001 to 50 wt%. In one embodiment, when the composition of the present invention is provided as a food or beverage, a pharmaceutical product, a quasi-pharmaceutical product, or the like, preferably, the amount of L-ergothioneine or a salt thereof is in the above ranges.

In the case of L-ergothioneine, the amount in terms of L-ergothioneine or an expression similar thereto refers to the amount of L-ergothioneine. In the case of a salt of L-ergothioneine, the amount in terms of L-ergothioneine or an expression similar thereto refers to a value obtained by multiplying the molar number of the salt by the molecular weight of L-ergothioneine.

The composition of the present invention can be fed or administered by a method appropriate to the composition form. In order to more sufficiently obtain the effect of the present invention, preferably, the composition of the present invention is orally fed (orally administered). The intake (administration amount) of the composition of the present invention is not limited as long as it is an amount that provides an anxiolytic effect, and may be appropriately set according to the administration form, administration method, body weight of a subject, and the like.

In one embodiment, when orally feeding or administering the composition of the present invention to a human (adult) as a subject, the intake of L-ergothioneine or a salt thereof in terms of L-ergothioneine per day is preferably 2 mg or more, more preferably 5 mg or more, still more preferably 10 mg or more, and is preferably 50 mg or less, more preferably 25 mg or less, still more preferably 20 mg or less. In one embodiment, when orally feeding or administering the composition of the present invention to a human (adult), the intake of L-ergothioneine or a salt thereof in terms of L-ergothioneine per day is preferably 2 to 50 mg, more preferably 5 to 25 mg, still more preferably 5 to 20 mg, particularly preferably 10 to 20 mg. Preferably, the above amount of the composition is fed or administered in one or more portions per day, for example, in one portion or several portions (for example, two or three portions) per day. In one embodiment of the present invention, the composition of the present invention may be an oral composition for feeding or administering the above amount of L-ergothioneine or a salt thereof per 60 kg body weight per day to a human.

In one embodiment, when the composition of the present invention is parenterally administered to a human (adult), the administration amount of L-ergothioneine or a salt thereof in terms of L-ergothioneine per day is, for example, preferably 2 to 50 mg, more preferably 5 to 25 mg, still more preferably 5 to 20 mg, particularly preferably 10 to 20 mg.

In one embodiment, in the case of a human (adult), preferably, the above amount of L-ergothioneine or a salt thereof per 60 kg body weight per day is fed or administered.

In one embodiment, preferably, the amount of L-ergothioneine or a salt thereof in the composition of the present invention per adult daily intake is 2 to 50 mg in terms of L-ergothioneine. The amount of L-ergothioneine or a salt thereof in the composition of the present invention per adult daily intake is more preferably 5 to 25 mg, still more preferably 5 to 20 mg, particularly preferably 10 to 20 mg in terms of L-ergothioneine.

Continuous feeding or administration of L-ergothioneine or a salt thereof is likely to result in a higher anxiolytic effect. Thus, in a preferred embodiment, the composition of the present invention is continuously fed or administered. In one embodiment of the present invention, the composition of the present invention is continuously fed or administered preferably for at least one week, more preferably for at least two weeks.

The subject to feeding or administration of the composition of the present invention (the subject to administration) is a human. The subject to feeding or administration of the composition of the present invention may be, for example, a human needing or wanting to prevent or reduce anxiety, a human needing or wanting to prevent or reduce anxiety symptoms caused by stress, a human feeling stressed, or the like. In one embodiment, the subject to administration may be a human with anxiety symptoms. In one embodiment, the subject to administration may be, for example, a human with symptoms such as decreased positive thinking, tension, nervousness, distress, mental agitation, or heartache. In one embodiment of the present invention, the subject to administration is preferably a human with temporary symptoms caused by stress, such as decreased positive thinking, tension, nervousness, distress, mental agitation, or heartache. The subject to administration may also be a human needing or wanting to prevent or reduce anxiety disorder or adjustment disorder (e.g., a human with anxiety disorder or a human with adjustment disorder), for example. In one embodiment, the subject to administration of the composition of the present invention may be a healthy person. The composition of the present invention can be used on a healthy person, for example, for purposes such as preventing anxiety symptoms and preventing a condition or disease which can be effectively prevented or ameliorated by preventing or reducing anxiety symptoms.

The anxiolytic composition of the present invention may be labeled with a function claim based on the anxiolytic action. The composition of the present invention may be labeled with one or more function claims such as "reducing anxiety", "reducing temporary mental stress", "lifting up a temporary depressed mood", "changing a temporary depressed mood into a positive mood", "making the mood lively", "changing a temporary depressed mood into a motivational mood", and "reducing a temporary decline in vigor and vitality".

In one embodiment of the present invention, preferably, the composition of the present invention is a food or beverage labeled with such a function claim. The label may be one indicating use of the composition for obtaining the function. The label may be directly attached to the composition or may be attached to a container or a package of the composition.

The present invention encompasses the following method and use:
a method of preventing or reducing an anxiety symptom, including feeding or administering L-ergothioneine or a salt thereof to a human; and
use of L-ergothioneine or a salt thereof for preventing or reducing a human anxiety symptom.

Feeding or administering L-ergothioneine or a salt thereof to a subject can prevent or reduce anxiety symptoms. Preferably, L-ergothioneine or a salt thereof is orally fed or administered to a human.

The method may be therapeutic or non-therapeutic. The use may be therapeutic or non-therapeutic.

In the method and use, L-ergothioneine or a salt thereof, preferred embodiments thereof, and the like are as described above for the anxiolytic composition of the present invention. In the method and use, preferably, L-ergothioneine or a salt thereof is fed or administered to a subject one or more times per day, for example, one to several times (e.g., two to three times) per day. In one embodiment, L-ergothioneine or a salt thereof can be used to prevent or reduce anxiety disorder, adjustment disorder, and the like.

In the method and use, L-ergothioneine or a salt thereof is simply used in an amount (effective amount) that provides the anxiolytic effect. Preferred administration amounts of L-ergothioneine or a salt thereof, preferred subjects to administration, and the like, are as described above for the anxiolytic composition of the present invention. L-ergothioneine or a salt thereof may be directly fed or administered or may be fed or administered as a composition containing L-ergothioneine or a salt thereof. For example, the composition of the present invention may be fed or administered.

L-ergothioneine or a salt thereof can be used to produce a food or beverage, a pharmaceutical product, a quasi-pharmaceutical product, or the like for use in preventing or reducing anxiety symptoms. In one embodiment, the present invention also encompasses use of L-ergothioneine or a salt thereof for producing an anxiolytic composition.

The present invention also encompasses L-ergothioneine or a salt thereof for use in preventing or reducing anxiety symptoms.

The description herein also includes the following anti-stress composition and method of preventing or reducing stress, for example:
an anti-stress composition for humans, containing L-ergothioneine or a salt thereof as an active ingredient;
a method of preventing or reducing stress, including administering L-ergothioneine or a salt thereof; and
use of L-ergothioneine or a salt thereof for preventing or reducing human stress.

The term "anti-stress" includes at least one of stress prevention and stress reduction. L-ergothioneine or a salt thereof has an action to reduce glutamate receptor activation as described above and thus can be used to prevent or reduce stress. The term "stress prevention" refers to reducing stress to experience to a level (degree) lower than the usual stress level. The term "stress reduction" encompasses reducing stress being experienced and relieving stress.

Stress may be mental stress. A preferred intake of L-ergothioneine or a salt thereof is the same as that of the anxiolytic composition. Preventing or reducing stress can contribute to preventing or ameliorating a condition or disease caused by stress.

### EXAMPLES

The present invention is described in further detail below with reference to examples, but the scope of the present invention is not limited thereto.

### <Example 1>

### (Evaluation of human anxiety symptoms)

In order to evaluate the influence of supplements containing ergothioneine on human anxiety symptoms and blood metabolome, a placebo-controlled, randomized, double-blind, parallel-group study was conducted on subjects feeling stressed daily, including male and female adults (46 subjects in a test food group; 46 subjects in a control food group; 92 subjects in total). In this study, one capsule containing 20 mg ergothioneine (test food) or one capsule not containing ergothioneine (control food) was fed per day for four weeks.

Anxiety symptoms were evaluated using State Trait Anxiety Inventory (STAI). The blood metabolome was measured by a capillary electrophoresis time-of-flight mass spectrometry (CE-TOF-MS) available from Human Metabolome Technologies, Inc. Anxiety symptoms of candidate subjects were evaluated by the State Trait Anxiety Inventory (STAI) before starting the test (before starting ingestion of the test food or control food), and those with higher values of state anxiety (the 92 subjects including adult males and females described above) were selected as subjects. The blood metabolome of each subject was measured for pre-testing. After ingestion of the test food or control food for four weeks, the anxiety symptoms and blood metabolome of each subject were measured again (testing on week 4).

### (Food for evaluation)

Two types of food for evaluation, which were indistinguishable in appearance, flavor, and the like, were used.
· Test food: capsule containing a test substance (20 mg L-ergothioneine)
· Control food: capsule containing no test substance

Each type of food for evaluation contained raw materials such as dextrin, hydroxypropyl cellulose, carrageenan, potassium chloride, and titanium oxide, in addition to the test substance. The control food was produced using the same raw materials used in the test food, except that the test substance (L-ergothioneine) was not added.

The State Trait Anxiety Inventory (STAI Y-1) has 20 items including, "I feel calm", "I feel secure", "I am tense", "I am strained ", "I feel at ease", "I feel upset", "I am presently worrying over possible misfortunes", "I feel satisfied", "I feel frightened", "I feel comfortable", "I feel self-confident", "I feel nervous", "I am jittery", "I feel indecisive", "I am relaxed", "I feel content", "I am worried", "I feel confused", "I feel steady", and "I feel pleasant", and each item is evaluated on a four-point scale from "Almost never" (one point) to "Almost always" (four points). The evaluation is performed by checking the items that the subject thinks are true.

An average state anxiety score (points) of the subjects in the test food group was regarded as the state anxiety score of the test food group. An average state anxiety score (points) of the subjects in the control food group was regarded as the state anxiety score (points) of the control food group. For each group, the amount of change in state anxiety score (Δ in state anxiety (points)) was determined by subtracting the state anxiety score at the time of pre-testing from the state anxiety score at the time of testing on week 4 ((score at the time of testing on week 4) - (score at the time of pre-testing)). The amount of change in state anxiety score (Δ in state anxiety (points)) was set to 0.

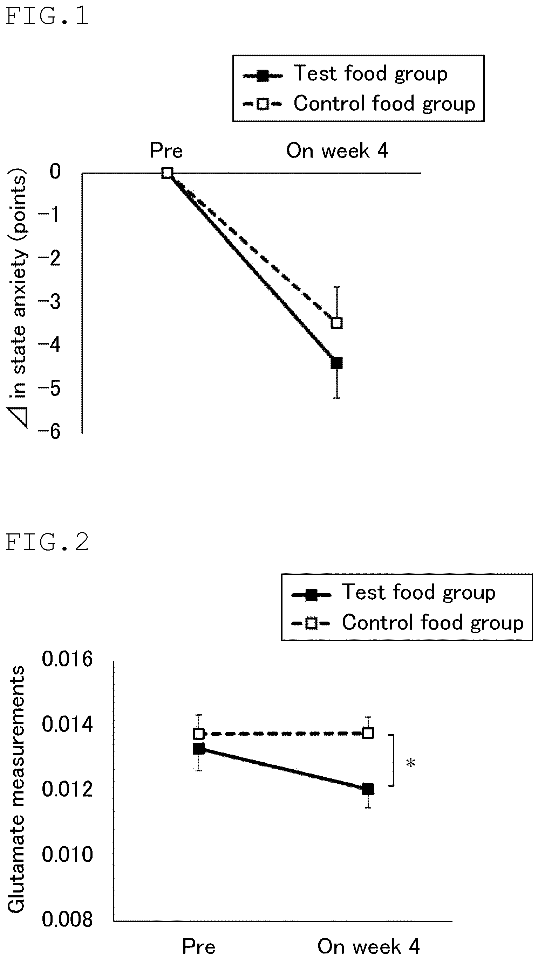

FIG. 1 is a graph showing changes in state anxiety score (Δ in state anxiety (points)) in the test food group and the control food group. The term "state anxiety" refers to temporary anxiety that arises in a particular situation. According to the results, the state anxiety score was more significantly reduced in the test food group than in the control food group.

In FIG. 1 and FIG. 2, ■ (black square) indicates the test food group, and □ (white square) indicates the control food group. On the horizontal axis, "Pre" indicates at the time of pre-testing and "On week 4" indicates at the time of testing on week 4.

The results showed that L-ergothioneine has an action to reduce temporary anxiety symptoms caused by mental stress because the state anxiety scores were reduced by L-ergothioneine. In the above evaluation, the anxiety symptoms that were evidently reduced by ingestion of L-ergothioneine include tension, fear, heartache, loneliness, decreased positive thinking, nervousness, distress, mental agitation, and the like.

FIG. 2 is a graph showing glutamate measurements identified by measuring the blood metabolome at the time of pre-testing and at the time of testing on week 4. Glutamate is an excitatory neurotransmitter in the central nervous system and is known to increase due to psychological stress. A 2-sample t-test (*: p < 0.05, vs. control food group) was performed for significance testing between the groups. According to the results, the blood glutamate concentration was significantly reduced in the test food group. This suggests that L-ergothioneine reduces the glutamate concentration to reduce the activation of the glutamate receptors and attenuate glutamate signaling, thereby showing the anxiolytic effect.

### <Example 2>

### (Evaluation of activity to inhibit binding of substrates to nicotinic acetylcholine receptors)

In order to evaluate molecular targets for reducing glutamate signaling by L-ergothioneine, nicotinic acetylcholine receptors associated with glutamate signaling were subjected to in vitro assay. The in vitro assay was commissioned to Eurofins Panlabs. The test was performed by the following method.

### Human α3β4 nicotinic acetylcholine receptors (α3β4 receptors)

Recombinant human α3/β4 nicotinic acetylcholine receptors (nAChR (α3β4)) expressed in Chinese hamster ovary-derived CHO-K1 cells were used. Cell membranes (0.6 µg) of the cells expressing nAChR (α3β4) were incubated in Tris-HCl buffer (pH 7.4) with 0.05 nM [125I]epibatidine and L-ergothioneine (L-ergothioneine concentration: 30 µM, 100 µM, 300 µM, 1000 µM, or 3000 µM) at 25°C for 60 minutes. Non-specific binding was estimated in the presence of 10 µM epibatidine. The cell membranes were filtered through a filter and washed, and the activity of L-ergothioneine to inhibit binding of substrates was evaluated using radioactivity of [125I]epibatidine as an index.

### Human α4β2 nicotinic acetylcholine receptors (α4β2 receptors)

Recombinant human α4/β2 nicotinic acetylcholine receptors (nAChR (α4β2)) expressed in human neuroblastoma-derived SH-SY5Y cells were used. Cell membranes (30 µg) of the cells expressing nAChR (α4β2) were incubated in Tris-HCl buffer (pH 7.4) with 0.6 nM [3H]cytisine and L-ergothioneine (L-ergothioneine concentration: 100 µM or 1000 µM) at 4°C for 120 minutes. Non-specific binding was estimated in the presence of 10 µM nicotine bitartrate. The cell membranes were filtered through a filter and washed, and the activity of L-ergothioneine to inhibit binding of substrates was evaluated using radioactivity of [3H]cytisine as an index.

### Human α7 nicotinic acetylcholine receptors (α7 receptors)

Recombinant human α7 nicotinic acetylcholine receptors (nAChR (α7)) expressed in human neuroblastoma-derived SH-SY5Y cells were used. Cell membranes (20 µg) of the cells were incubated in phosphate buffer (pH 7.4) with 0.4 nM [125I]-α-bungarotoxin and L-ergothioneine (L-ergothioneine concentration: 100 µM or 1000 µM) at 37°C for 120 minutes. Non-specific binding was estimated in the presence of 1 µM α-bungarotoxin. The cell membranes were filtered through a filter and washed, and the activity of L-ergothioneine to inhibit binding of substrates was evaluated using radioactivity of [125I]-α-bungarotoxin as an index.

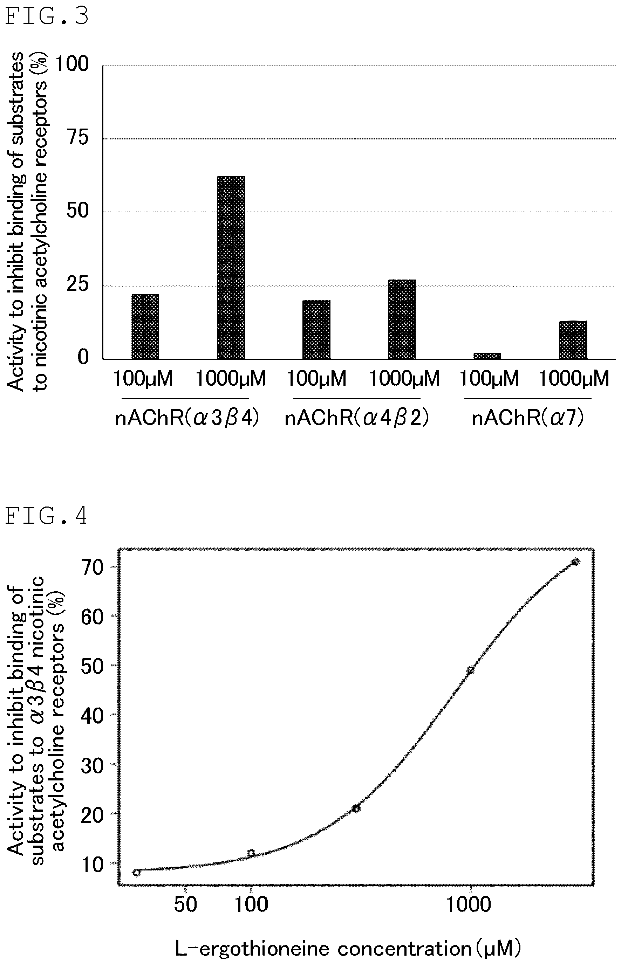

FIG. 3 shows results of the evaluation of the activity of L-ergothioneine to inhibit binding of substrates to the nicotinic acetylcholine receptors (nAChR). The concentration (µM) on the horizontal axis is the concentration of L-ergothioneine. L-ergothioneine showed the activity to inhibit binding of the substrates to the nicotinic acetylcholine receptors, i.e., α3β4 receptors, α4β2 receptors, and α7 receptors. L-ergothioneine was confirmed to have a strong activity to inhibit binding of substrates particularly to α3β4 (nAChR (α3β4)) among the nicotinic acetylcholine receptors.

FIG. 4 shows results of the evaluation of the activity of L-ergothioneine to inhibit binding of substrates to the α3β4 nicotinic acetylcholine receptors based on five concentrations. L-ergothioneine showed the activity in a concentration-dependent manner, and the 50% inhibitory concentration was 1.11 mM. α3β4 nicotinic acetylcholine receptors are expressed in the brain, and inhibition of the receptors is known to reduce glutamate signaling. Thus, inferentially, L-ergothioneine attenuated glutamate signaling also by inhibiting the nicotinic acetylcholine receptors such as α3β4 nicotinic acetylcholine receptors, showing the anxiolytic effect.

## Claims

1. An anxiolytic composition for humans, comprising
L-ergothioneine or a salt thereof as an active ingredient.

2. The composition according to claim 1,
wherein the composition reduces glutamate receptor activation.

3. The composition according to claim 1 or 2,
wherein the composition is an oral composition.

4. The composition according to any one of claims 1 to 3,
wherein the composition is a food or beverage.

5. The composition according to any one of claims 1 to 4,
wherein an amount of L-ergothioneine or a salt thereof in the composition per adult daily intake is 2 to 50 mg in terms of L-ergothioneine.

6. A method of preventing or reducing an anxiety symptom, comprising
administering L-ergothioneine or a salt thereof to a human.

7. Use of L-ergothioneine or a salt thereof for preventing or reducing a human anxiety symptom.
